# EUROPÄISCHE PATENTSCHRIFT

(11) **EP 0 502 349 B1**
(45) Veröffentlichungstag und Bekanntmachung des Hinweises auf die Patenterteilung: **02.08.1995**
(21) Anmeldenummer: 92102483.2
(22) Anmeldetag: 14.02.1992
(51) Int. Cl.: B22C 9/04, A61F 2/30, A61L 27/00

(54) **Verfahren zur Herstellung eines Implantates mit einer seine Oberfläche zumindest teilweise bedeckenden metallischen offenzelligen Struktur**
Process of manufacturing an implant with a metallic open-cell structuure at least partially covering its surface
Procédé pour fabriquer un implant avec une structure métallique à pores ouverts recouvrant au moins partiellement sa surface

(30) Priorität: 05.03.1991 DE 4106971
(43) Veröffentlichungstag der Anmeldung: 09.09.1992
(73) Patentinhaber: ESKA Implants GmbH & Co., 23556 Lübeck (DE)
(72) Erfinder: Ahlers, Olaf, W-2000 Hamburg 70 (DE)
(74) Vertreter: Fuchs Mehler Weiss

(56) Entgegenhaltungen:
- EP-A- 0 399 163
- EP-A- 0 415 872
- WO-A-83/03346
- DE-U- 9 011 363
- FR-A- 2 350 827
- FR-A- 2 356 465

## Beschreibung

Die Erfindung betrifft ein Verfahren zur Herstellung eines Implantates mit einer seine Oberfläche zumindest teilweise bedeckenden metallischen offenzelligen Struktur gemäß dem Oberbegriff des Anspruchs 1.

Die damit hergestellten Implantate ermöglichen das Einwachsen von Knochenmaterial in die offenzellige Oberflächenstruktur, wodurch für deren dauerhafte Fixation im Knochen gesorgt wird.

Ein ähnliches Verfahren ist bekannt aus der DE-OS 27 30 004. Darin wird insbesondere vorgeschlagen, als Partikel zur Erzeugung einer rauhen Oberfläche zunächst des Positivmodells und schließlich des mit dem Verfahren hergestellten Implantats als metallisches Abbild dieses Modells Partikel in Form von Kügelchen mit dem Basismodell zu verhaften.

Das Implantat weist an den Stellen rauher Oberfläche erhabene kugelsegmentartige Erhebungen mit Hinterschnitten auf. An diese Oberflächen kann nach der Implantation Knochenmaterial heranwachsen und soll zur Dauerfixation des Implantats am Knochen dienen.

Wenn dies auch in der Praxis gelingen mag, zeigen die nach dem erwähnten Verfahren hergestellten Implantate folgende Nachteile auf:
Das Heranwachsen von Knochenmaterial ist zwar möglich, jedoch haben medizinische Untersuchungen Grenzschichtprobleme beleuchtet, denen zufolge eine Versorgung des Knochens an den Schichten hin zum Implantat aufgrund eines fehlenden Substratflusses nicht erfolgt, so daß die dauerhafte knöcherne Fixation des Implantats in Frage gestellt ist.

Darüberhinaus stellt sich die Oberfläche des Implantats immer noch recht glatt dar, wodurch die Primärstabilität des Implantats direkt nach der Implantation relativ gering ist.

Hinter dem erwähnten Verfahren steht die medizinische Philosophie, daß ein Implantat bei einem notwendig werdenden Revisionseingriff, etwa infolge einer Infektion, entfernt werden können sollte, ohne dabei den das Implantat umgebenden Knochen zerstören zu müssen.

In den vergangenen Jahren ist aber eine Operationstechnik entwickelt worden, die einen Revisionseingriff zur Entfernung von mit Knochenmaterial durchwachsenen Implantaten (DE 32 24 265 A1) gestattet, bei der der Knochen gespalten, das Implantat entfernt, ein neues Implantat eingesetzt und die Knochenhälften zusammengefügt werden und diese schließlich wieder miteinander verwachsen.

Gemäß der vorerwähnten Druckschrift wird vorgeschlagen, ein Implantat nach der üblichen Feingußtechnik herzustellen, wobei als Positivmodell ein offenporiger Kunststofformkörper wie beispielsweise ein Filterschaum verwendet wird. Dabei ist zu beachten, daß die Stärke der die Zellen einfassenden Stege eine Funktion der Zellenweite ist. Dies ist herstellungsbedingt bei der Erzeugung derartiger Schäume und kann nicht gesteuert werden.

Es hat sich gezeigt, daß die Stege bei Anwendung der Gußstücke als Implantate zu dünn sind, weswegen in der genannten Druckschrift deren Verstärkung mittels flüssigem Wachs oder mittels einer Wachs-Wasser-Emulsion vorgeschlagen wurde.

Dieses Auftragverfahren ist relativ schwer handhabbar. Darüberhinaus zeigt der Filterschaum trotz globaler relativer Formkonstanz lokale Eigenheiten wie beispielsweise kleinere Zellen auf. Dieser Umstand macht sich bei der Durchführung des Gießverfahrens nach dem Erstarren des in die Hohlräume gegossenen Metalls negativ bemerkbar bei der Entfernung der keramischen Einbettmasse. Bedenkt man, daß fertige Implantate von höchster Reinheit sein müssen, um implantiert werden zu können, gestaltet es sich als äußerst schwierig, die letzten Reste der Einbettmasse vom Gußstück, insbesondere aus zufälligerweise kleineren Zellen in der räumlichen Tiefe zu entfernen.

Ein ebenfalls unter Anwendung der Feingießtechnik hergestelltes Prothesenteil zeigt die DE 90 11 363.2 U1. Als Positivmodell wird ein Grundkörper mit einem damit verbundenen Gitterelement, vorzugsweise aus Wachs, verwendet. Das Gitterelement soll durch ein Spritzgußverfahren hergestellt sein und wird wie eine Matte um den Grundkörper gewickelt. Hierzu muß das Gitterelement elastisch genug sein, da es ansonsten brechen würde. Wohl aus diesem Grunde sind die Stege des Gitterelementes in ihrer Dicke mit vorzugsweise 0,05 cm angegeben. In vergleichbarer Größenordnung liegen dann selbstverständlich die Stege der gitterförmigen Implantatoberfläche nach Durchführung des Gießverfahrens. Sollen die Stege eine größere Dicke aufweisen, müßte auf ein Auftragverfahren der vorstehend beschriebenen Art zurückgegriffen werden.

Weitere Verfahren sind in der DE 39 17 033 C1 und DE 39 28 394 C2 beschrieben. Darin wird vorgeschlagen, die Stege eines offenporigen Kunststoffträgers (Filterschaum), welcher auf einem Wachsgrundkörper verhaftet ist und mit disem zusammen das Positivmodell des herzustellenden Gußstückes (Implantat) bildet, zu verstärken durch ein Auftragen eines Zweikomponenten-Silikons (DE 39 17 033 C1) bzw. von Polymethylmethacrylat oder Polyesterharz (DE 39 28 394 C2).

Aufgabe der vorliegenden Erfindung ist es, ein Verfahren der eingangs erwähnten Art so weiterzubilden, daß die mit ihm erzeugten Oberflächenstrukturen des Implantats diskret und so stabil sind, daß es einer weiteren Behandlung des Positivmodells nicht bedarf, und daß Schwierigkeiten beim Entfernen der keramischen Einbettmasse nach dem Gießvorgang aufgrund von lokalen Abweichungen der Strukturen nicht auftreten.

Gelöst wird diese Aufgabe durch das Verfahren mit den Merkmalen des Anspruchs 1.

Als kennzeichnend wird hierbei die Form der auf das Basismodell aufzubringenden Partikel angesehen. Diese weisen einen kugelförmigen, doppelkegel- oder doppelpyramidenförmigen o.ä. Grundkörper mit von diesem radial abgehenden vier bis acht, vorzugsweise sechs Zapfen auf, von denen mindestens drei mit dem Basismodell verhaftet werden. Der Zwischenraum zwischen den mindestens drei zum Basismodell verhafteten Zapfen bildet sich nach Durchführung des Verfahrens als offene Zelle der Oberflächenstruktur im Implantat ab, durch welche Knochenmaterial unter Aufrechterhaltung des lebensnotwendigen Substratsflusses durchwächst.

Die Partikel eines Positivmodells weisen alle dieselben Partikel auf, die beispielsweise durch ein Spritzgußverfahren hergestellt werden können. Diese globale Formkonstanz bringt eine erhebliche Vereinfachung bei der Entfernung der keramischen Einbettmasse nach dem Gießvorgang mit sich.

Jedes der Partikel bildet sich nach der Durchführung des Verfahrens als einstückiger Bestandteil des Implantats ab, wobei als weiterer gewichtiger Vorteil die hohe Maßgenauigkeit des Implantats zu erwähnen ist. Etwaige Zusatzmaßnahmen zur Erhöhung der Stabilität der Zapfen, beispielsweise durch Auftragungsverfahren, sind nicht erforderlich, da die Dicke der Zapfen der Partikel von vornherein während deren Herstellungsprozesse gezielt gesteuert werden kann. Bevorzugt werden Zapfendicken im Bereich von 0,6 bis 1,6 mm.

Vorteilhaft sind die verwendeten Partikel von symmetrischer Formgestalt und weisen sechs äquidistant auf der Oberfläche des kugeligen, doppelkegel- oder doppelpyramidenförmigen Grundkörpers verteilte, strahlenförmig von diesem abgehende Zapfen auf. Bildlich läßt sich ein solches Partikel als ein Kristallpunkt eines Kristallgitters vorstellen, bei dem der Grundkörper die Stelle eines Atoms und die Zapfen die Bindungen an ein benachbartes Atom einnehmen.

Im vorbeschriebenen Fall werden die Enden dreier Zapfen mit dem Basismodell verhaftet, die anderen drei ragen von dem Basismodell weg und sorgen beim fertigen Implantat für eine extreme Oberflächenaggressivität in dem Sinne, daß für eine hervorragende Primärstabilität und für eine extreme Reizung des umgebenden Knochens gesorgt ist.

Letzteres stimuliert den Knochen zum schnellen Durchwachsen der Oberflächenstruktur.

Das Endmaß des herzustellenden Implantats kann unter Verwendung eines standardisierten Basismodells dadurch variiert werden, daß mehrere Schichten von Partikeln an dem Positivmodell verhaftet werden.

In diesem Falle stellt sich der Partikelaufbau bildlich wie der Aufbau eines Kristallgitters dar. Sämtliche vorerwähnten Vorteile bei der Durchführung des geschilderten Verfahrens bleiben erhalten.

Damit bei der Erhitzung des Modells auch die Partikel rückstandsfrei entfernt werden können, bestehen diese aus einem der folgenden Stoffe:
Wachs, Polypropylen, Polyäthylen, Polymethylmetacrylat oder jedem anderen geeigneten Stoff.

Die Erfindung wird anhand eines Ausführungsbeispiels eines Positivmodells eines Implantates erläutert.

Es zeigen hierbei:
- Figur 1:: Die Ansicht eines Positivmodells für einen Hüftgelenkschaft
- Figur 2:: die vergrößerte Einzelheit Z in Figur 1.

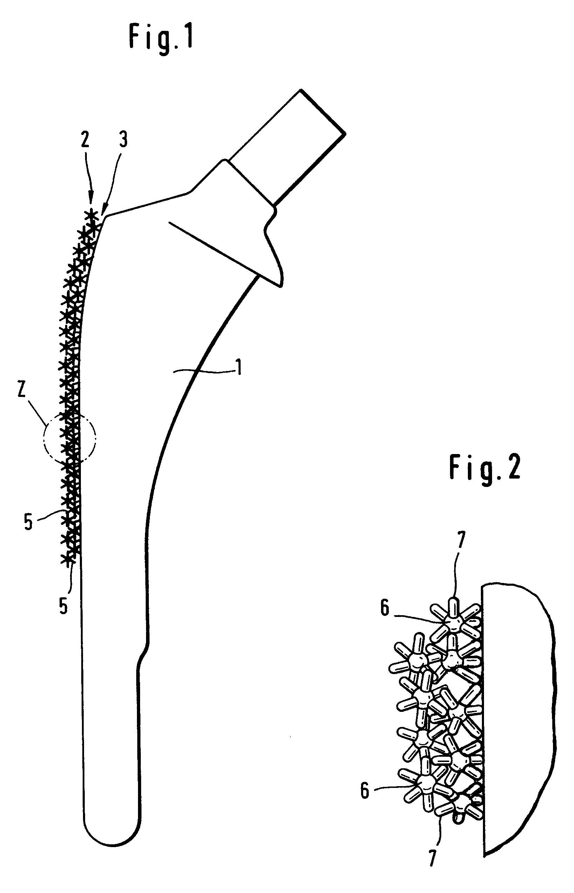

Figur 1 zeigt die Ansicht eines in dem erfindungsgemäßen Verfahren verwendeten Positivmodells eines Hüftgelenksstiels. Das Positivmodell besteht aus einem Basismodell 1 sowie aus mit diesem verhafteten Partikeln 5. Im gezeigten Ausführungsbeispiel sind zwei Schichten 2 und 3 von Partikeln 5 dargestellt. Der Bereich des Basismodells 1, welcher mit den Partikeln 5 versehen ist, ist nur beispielhaft dargestellt.

Einzelheiten der partikelanordnung auf dem Basismodell 1 zeigt die Figur 2, welche die Vergrößerung der Einzelheit Z in Figur 1 darstellt.

Deutlich erkennbar ist die hier verwendete Form der Partikel. Diese weisen einen kugeligen Grundkörper 6 auf, von dem aus gleichmäßig über seine Oberfläche verteilt sechs Zapfen 7 strahlenförmig ausgehen. Vorliegend sind zwei Schichten von Partikeln übereinander geschichtet. Die äußere Schicht ist mit der zuunterst am Basismodell 1 anhaftenden Schicht verkeilt.

Nach dem Gießen stellt sich das metallische Abbild entsprechend dem Positivmodell dar. Es entsteht bei der Ausbildung des Positivmodells gemäß der Figuren ein Implantat mit einer offenzelligen Raumnetzstruktur an Bereichen seiner Oberfläche. Aufgrund der Konstanz der offenen Zellen ist eine Reinigung von der keramischen Einbettmasse ohne Probleme möglich.

## Patentansprüche

1. Verfahren zur Herstellung eines Implantates mit zumindest teilweise strukturierter offenzelliger Oberfläche unter Anwendung eines Positivmodells, das aus einem veraschbaren oder schmelzbaren Basismodell sowie aus mit diesem verhafteten veraschbaren oder schmelzbaren Partikeln besteht, die die später strukturierten Oberflächenbereiche des Implantates bedecken, bei dem das Positivmodell als Ganzes in eine keramische Einbettmasse eingebettet wird, das Modell erhitzt wird, wobei das Basismodell verascht oder schmilzt, die Partikel veraschen oder schmelzen und die keramische Einbettmasse gebrannt wird, in die verbleibenden Hohlräume geschmolzenes Material gefüllt wird und nach dessen Erstarren die keramische Einbettmasse entfernt wird,
dadurch gekennzeichnet,
daß die Partikel (5) einen Grundkörper (6) mit von diesem radial abgehenden vier bis acht Zapfen (7) aufweisen, von denen wenigstens drei mit dem Basismodell (1) verhaftet werden.

2. Verfahren nach Anspruch 1, dadurch gekennzeichnet, daß die Partikel (5) von symmetrischer Formgestalt sind und sechs äquidistante, strahlenförmig von ihrem Grundkörper (6) abgehende Zapfen (7) aufweisen.

3. Verfahren nach Anspruch 1 oder Anspruch 2, dadurch gekennzeichnet, daß der Grundkörper (6) eines Partikels (5) einen Durchmesser von 1,5 bis 3 mm aufweist und die Länge der von ihm abgehenden Zapfen (7) 0,8 bis 1,6 mm beträgt.

4. Verfahren nach ein dem der Ansprüche 1 bis 3, dadurch gekennzeichnet, daß mehrere Schichten (2, 3) von Partikeln (5) an dem Positivmodell verhaftet werden.

5. Verfahren nach Anspruch 4, dadurch gekennzeichnet, daß bis zu vier Schichten von Partikeln übereinander verhaftet werden.

6. Verfahren nach einem der Ansprüche 1 bis 5, dadurch gekennzeichnet, daß die Partikel (5) aus Wachs bestehen.

7. Verfahren nach einem der Ansprüche 1 bis 5, dadurch gekennzeichnet, daß die Partikel (5) aus Polypropylen bestehen.

8. Verfahren nach einem der Ansprüche 1 bis 5, dadurch gekennzeichnet, daß die Partikel (5) aus Polyäthylen bestehen.

9. Verfahren nach einem der Ansprüche 1 bis 5, dadurch gekennzeichnet, daß die Partikel (5) aus Polymethylmetacrylat bestehen.

## Claims

1. Process for producing an implant having an at least partly structured open-pore surface using a positive model which consists of base model which is capable of being incinerated or melted, and of particles which are capable of being incinerated or melted adhered to the latter, which cover the surface regions of the implant which are structured later, in which the entire positive model is embedded in a ceramic embedding composition, the model is heated, wherein the base model is incinerated or melts, the particles are incinerated or melt and the ceramic embedding composition is fired, molten material is poured into the remaining cavities and after it has solidified, the ceramic embedding composition is removed, characterised in that the particles (5) have a base (6) with four to eight pins (7) emerging radially from the latter, at least three of which are adhered to the base model (1).

2. Process according to claim 1, characterised in that the particles (5) have symmetrical shape and six equidistant pins (7) emerging like rays from their base (6).

3. Process according to claim 1 or claim 2, characterised in that the base (6) of a particle (5) has a diameter of 1.5 to 3 mm and the length of the pins (7) emerging from it is 0.8 to 1.6 mm.

4. Process according to one of claims 1 to 3, characterised in that several layers (2, 3) of particles (5) are adhered to the positive model.

5. Process according to claim 4, characterised in that up to four layers of particles are adhered one above another.

6. Process according to one of claims 1 to 5, characterised in that the particles (5) consist of wax.

7. Process according to one of claims 1 to 5, characterised in that the particles (5) consist of polypropylene.

8. Process according to one of claims 1 to 5, characterised in that the particles (5) consist of polyethylene.

9. Process according to one of claims 1 to 5, characterised in that the particles (5) consist of polymethylmethacrylate.

## Revendications

1. Procédé pour fabriquer un implant avec au moins une surface à cellule ouverte partiellement structurée en utilisant un modèle positif, qui se compose d'un modèle de base qui peut être incinéré ou fondu ainsi que de particules qui peuvent être incinérées ou fondues attachées à celui-ci, qui recouvrent les régions de surfaces structurées ultérieurement de l'implant, dans lequel le modèle positif est noyé en totalité dans un noyau céramique, le modèle est chauffé, le modèle de base étant incinéré ou fondu, les particules sont incinérées ou fondent et le noyau céramique est cuit, les cavités restantes sont chargées du matériau fondu et le noyau de céramique est enlevé après solidification de celui-ci, caractérisé en ce que, les particules (5) présentent un corps de base (6) avec quatre à huit pics (7) partant radialement de celui-ci, dont au moins trois sont attachés au modèle de base (1).

2. Procédé selon la revendication 1, caractérisé en ce que les particules (5) sont de conception symétrique et présentent six pics équidistants (7) partant radialement du corps de base (6).

3. Procédé selon la revendication 1 ou 2, caractérisé en ce que le corps de base (6) d'une particule (5) présente un diamètre compris entre 1,5 et 3 mm et la longueur des pics (7) en partant fait de 0,8 à 1,6 mm.

4. Procédé selon l'une quelconque des revendications 1 à 3, caractérisé en ce que plusieurs couches (2, 3) de particules (5) sont attachées au modèle positif.

5. Procédé selon la revendication 4, caractérisé en ce que jusqu'à quatre couches de particules peuvent être attachées l'une sur l'autre.

6. Procédé selon l'une quelconque des revendications 1 à 5, caractérisé en ce que les particules (5) se composent de cire.

7. Procédé selon l'une quelconque des revendications 1 à 5, caractérisé en ce que les particules (5) se composent de polypropylène.

8. Procédé selon l'une quelconque des revendications 1 à 5, caractérisé en ce que les particules (5) se composent de polyéthylène.

9. Procédé selon l'une quelconque des revendications 1 à 5, caractérisé en ce que les particules (5) se composent de polyméthacrylate de méthyle.
